# Europäisches Patentamt
## European Patent Office
## Office européen des brevets

(19)

(11) Veröffentlichungsnummer: **0 065 125**
**B1**

(12) # EUROPÄISCHE PATENTSCHRIFT

(45) Veröffentlichungstag der Patentschrift:
31.10.84

(51) Int. Cl.³: **C 07 C 43/225, C 08 K 5/06**

(21) Anmeldenummer: 82103486.5

(22) Anmeldetag: 24.04.82

(54) **1,4-Bis-(4-chlor-2-methoxystyryl)-benzol sowie dessen Verwendung als Weisstöner für makromolekulare organische Stoffe.**

(30) Priorität: 09.05.81 DE 3118525

(43) Veröffentlichungstag der Anmeldung:
24.11.82 Patentblatt 82/47

(45) Bekanntmachung des Hinweises auf die Patenterteilung:
31.10.84 Patentblatt 84/44

(84) Benannte Vertragsstaaten:
CH DE FR GB LI

(56) Entgegenhaltungen:
FR - A - 1 415 977
FR - A - 1 576 018

(73) Patentinhaber: BAYER AG, Konzernverwaltung RP
Patentabteilung, D-5090 Leverkusen 1 Bayerwerk (DE)

(72) Erfinder: Schellhammer, Carl-Wolfgang, Dr.,
Katharinental 26, D-5060 Bergisch Gladbach 2 (DE)
Erfinder: Wehling, Bernhard, Dr.,
Andreas-Gryphius-Strasse 26, D-5000 Köln 80 (DE)

## Beschreibung

Es ist bekannt, daß Verbindungen der Bisstyrylbenzolreihe wertvolle Weißtöner für verschiedenartige makromolekulare Materialien sind (vgl. DE-PS 1 273 812).

Allerdings genügen die zahlreichen beispielhaft in der Literatur beschriebenen Verbindungen dieser Substanzklasse nicht immer den anwendungstechnischen Anforderungen, die heute an hochwertige Weißtöner gestellt werden. Dies gilt insbesondere für spezielle Verwendungszwecke, wie etwa das Massefärben von Thermoplasten.

Es wurde nun gefunden, daß 1,4-Bis-(4-chlor-2-methoxystyryl)-benzol aus der Vielzahl der bekannten Bisstyrylbenzole durch ein besonderes interessantes Eigenschaftsbild herausragt. Hervorzuheben ist vor allen die Eignung dieser Verbindung zum Weißtönen von thermoplastischen Materialien, wobei sie sich unter anderem durch gute Lichtechtheit, gute Ausgiebigkeit und eine angenehme Farbnuance auszeichnen.

Geeignete thermoplastische Stoffe sind beispielsweise: Polyester, Vinylpolymere, Acrylpolymere, Allylpolymere, Polyamide, Polyolefine sowie andere handelsübliche Polymerisate und polykondensate.

Außer diesen vollsynthetischen Polymeren kommen auch natürliche bzw. halbsynthetische Produkte in Betracht, wie z. B. Zellglas, Celluloseacetat und Cellulosebutyrat sowie deren Mischungen mit den zuvor erwähnten Syntheseprodukten.

Weiterhin eignet sich der neue Weißtöner zum optischen Aufhellen von nicht- bzw. wenig verfärbten Duroplasten, wie z. B. Melaimharzen.

Ganz besonders geeignet ist die neue Bisstyrylbenzolverbindung zum Weißtönen von PVC weich, Polystyrol, ABS-Polymerisaten, Polyethylen und Polypropylen.

Der Weißtöner kann in verschiedener Weise diesen makromolekularen Stoffen zugesetzt werden.

Beispielsweise kann er Spinnschmelzen und Spinnlösungen von faden- und filmbildenden Polymeren zugemischt werden.

Bisweilen ist es vorteilhafter, den Weißtöner vor der Poylmerisation den polymerisier- oder polykondensierbaren Monomeren oder den Vorkondensaten beizumischen.

Der Weißtöner kann aber auch nachträglich in die makromolekularen Stoffe in üblicher Weise eingearbeitet werden.

Die eingesetzten Mengen betragen 0,001—0,1 Gewichtsprozent, bezogen auf das aufzuhellende Material.

Die Herstellung des Weißtöners kann nach verschiedenen an sich bekannten Verfahren erfolgen (z. B. DE-PS 1 108 219 und 1 112 072 sowie GB-PS 1 043 501).

Ein Verfahren ist dadurch gekennzeichnet, daß man 2 Mol eines 4-Chlor-2-methoxybenzyl-phosphonsäuredialkylester (Alkyl z. B. Methyl oder Ethyl) mit Terephthalaldehyd kondensiert.

Bei einem anderen Verfahren wird 4-Chlor-2-methoxybenzaldehyd (2 Mol) mit p-Xylylen-$\alpha,\alpha'$-bisphosphonsäuredialkylester umgesetzt.

Bei beiden Methoden arbeitet man zweckmäßigerweise in einem geeigneten organischen Lösungsmittel, wie z. B. Dimethylformamid, in Gegenwart eines alkalischen Kondensationsmittels (z. B. KOH oder $NaOCH_3$).

### Herstellungsbeispiel

Durch Bromierung von 4-Chlor-2-methoxytoluol mit N-Bromsuccinimid in siedendem Tetrachlorkohlenstoff in Gegenwart von Dibenzoylperoxid erhält man 4-Chlor-2-methoxybenzylbromid als farblose Flüssigkeit vom $Kp_{13} = 140—145°$ C. Durch Umsetzung mit Triethylphosphit in siedendem Xylol entsteht daraus 4-Chlor-2-methoxybenzylphosphonsäurediethylester; es handelt sich dabei um eine farblose, viskose Flüssigkeit vom $Kp_{0,1} = 132°$ C.

Zu einer Mischung aus 29,3 g (0,1 Mol) 4-Chlor-2-methoxybenzylphosphonsäurediethylester, 6 g (0,049 Mol) Terephthalaldehyd und 100 ml N,N-Dimethylformamid tropft man unter Stickstoff 36 g (0,2 Mol) 30%ige Natriummethylatlösung. Man rührt die Mischung 1 Stunde bei Raumtemperatur und 3 Stunden bei 60° C. Man gießt den Kolbeninhalt dann auf Eiswasser, neutralisiert, saugt das abgeschiedene Material ab und trocknet es nach dem Waschen mit Wasser. Man erhält 18 g gelbes Rohprodukt, das aus Xylol/Tonsil umgelöst wird. Das 1,4-Bis-(4-chlor-2-methoxystyryl)-benzol liegt dann in Form von gelben Nädelchen vor, die bei 188—192° C schmelzen. Eine Lösung der Substanz in N,N-Dimethylformamid zeigt bei 372,9 nm ein Absorptionsmaximum, die molare Extinktion liegt bei 60 000.

### Anwendungsbeispiele

### Beispiel 1

102 Teile einer Polyvinylchloridmasse, bestehend aus 70 Teilen Polyvinylchlorid, 30 Teilen eines Weichmachers, z. B. Dioctylphthalat, und 2 Teilen eines Stabilisators werden mit 0,01 Teilen des im Herstellungsbeispiel genannten Weißtöners ca. 5 Minuten bei 150° C auf dem Zweiwalzenstuhl gewalzt und zu Folien ausgezogen. Zur Herstellung von gedeckten Folien werden der Masse vor dem Walzen 2,5 Teile Titandioxid zugesetzt. Man erhält auf diese Weise vorzüglich weißgetönte PVC-Weichfolien.

### Beispiel 2

0,05 Teile des im Herstellungsbeispiel genannten Weißtöners werden mit 100 Teilen eines Polystyrol-Granulates mit einem Titandioxid-Gehalt von 2% vermischt und bei 230°C auf einer Schneckenspritzgießmaschine zu Musterplättchen verspritzt. Die erhaltenen Plätchen zeigen einen ausgezeichneten Aufhelleffekt.

### Beispiel 3

Ersetzt man das Polystyrol-Granulat durch ein ABS-Granulat mit einem Titandioxidgehalt von 4% und verfährt im übrigen wie im Anwendungsbeispiel 2 angegeben, so erhält man hervorragend weißgetönte ABS-Musterplättchen.

### Beispiel 4

Ersetzt man das Polystyrol-Granulat durch ein Polypropylen-Homopolymerisat mit einem Titandioxid-Gehalt von 2% und verfährt im übrigen wie in Anwendungsbeispiel 2 beschrieben, so erhält man PP-Musterplättchen, die einen hervorragenden Weißeffekt zeigen.

**Patentansprüche**

1. 1,4-Bis-(4-chlor-2-methoxystyryl-benzol.
2. Verwendung von 1,4-Bis-(4-chlor-2-methoxystyryl)-benzol zum Weißtönen von makromolekularen organischen Stoffen.
3. Verwendung von 1,4-Bis-(4-chlor-2-methoxystyryl)-benzol zum Weißtönen von Thermoplasten.

**Claims**

1. 1,4-Bis-(4-chloro-2-methoxystyryl)-benzene.
2. Use of 1,4-bis-(4-chloro-2-methoxystyryl)-benzene for whitening macromolecular organic substances
3. Use of 1,4-bis-(4-chloro-2-methoxystyryl)-benzene for whitening thermoplastics.

**Revendications**

1. Le 1,4-bis-(4-chloro-2-méthoxystyryl)-benzène.
2. L'utilisation du 1,4-bis-(4-chloro-2-méthoxystyryl)-benzène pour l'azurage de matières organiques macromoléculaires.
3. L'utilisation du 1,4-bis-(4-chloro-2-méthoxystyryl)-benzène pour l'azurage des matières thermoplastiques.